# EUROPEAN PATENT SPECIFICATION

(11) **EP 2 460 784 B1**
(45) Date of publication and mention of the grant of the patent: **17.06.2020**
(21) Application number: 10804526.1
(22) Date of filing: 29.07.2010
(51) Int. Cl.: C07C 1/24, C07C 11/06, C07B 61/00, B01J 29/035, B01J 29/40, B01J 29/90, B01J 27/232, B01J 37/04, B01J 37/10, B01J 38/14

(54) **METHOD FOR PRODUCING PROPYLENE AND CATALYST FOR PRODUCING PROPYLENE**
VERFAHREN ZUR PROPYLENHERSTELLUNG UND KATALYSATOR ZUR PROPYLENHERSTELLUNG
PROCÉDÉ DE PRODUCTION DU PROPYLÈNE ET CATALYSEUR DE PRODUCTION DU PROPYLÈNE

(30) Priority: 30.07.2009 JP 2009177551
(43) Date of publication of application: 06.06.2012
(73) Proprietor: Mitsubishi Chemical Corporation, Tokyo 100-8251 (JP); JGC Corporation, Nishi-ku Yokohama 220-6001 (JP)
(72) Inventor: YAMAGUCHI, Masashi, Yokohama-shi, Kanagawa 227-8502 (JP); YOSHIKAWA, Yumiko, Yokohama-shi, Kanagawa 227-8502 (JP); IWADE, Shinji, Kurashiki-shi, Okayama 712-8054 (JP); SETOYAMA, Tohru, Yokohama-shi, Kanagawa 227-8502 (JP); OKITA, Atsushi, Ibaraki 311-1313 (JP); HONDA, Kazunori, Ibaraki 311-1313 (JP); INAKI, Chizu, Ibaraki 311-1313 (JP); KUSANAGI, Yusuke, Ibaraki 311-1313 (JP)
(74) Representative: Vossius & Partner Patentanwälte Rechtsanwälte mbB
(86) International application number: PCT/JP2010/062855
(87) International publication number: WO 2011/013780

(56) References cited:
- EP-A1- 0 921 181
- EP-A1- 1 508 555
- EP-A1- 2 082 802
- WO-A1-2008/035743
- JP-A- 61 097 231
- JP-A- 2007 297 363
- JP-A- 2008 074 764
- JP-A- 2008 080 301
- US-A- 4 504 690
- US-A- 4 579 994

## Description

### [Technical Field]

This invention relates to a method for manufacturing propylene, comprising bringing an oxygen-containing compound into contact with a catalyst in the presence of an olefin having 4 or more carbon atoms, and a catalyst to be used therein.

### [Background of the Invention]

As the method for manufacturing propylene, steam cracking of naphtha and fluidized catalytic cracking of vacuum gas oil have been conventionally and generally carried out, and an MTO (methanol to olefin) process using at least one of methanol and dimethyl ether as the raw material is drawing attention in recent years.

In addition, as a mode of the MTO process, it is also known an MTP (methanol to propylene) process in which propylene is selectively manufactured by recycling the formed olefins other than propylene into the reactor (e.g., Patent Reference 1). In the MTP process, zeolite (MFI type or the like) is generally used as the catalyst.

In the MTO process, the reaction efficiency is lowered due to deactivation of the zeolite catalyst with time. As the main cause of this deactivation of zeolite catalyst, there may be mentioned deposition of carbon component (coke) on the zeolite catalyst by the reaction, and elimination of aluminum from the zeolite framework (dealumination) due to contact with the steam formed by the reaction.

Accordingly, various examinations have been made on the method for reducing such catalyst deactivation. Patent Reference 2 describes that a catalyst manufactured by mixing zeolite having a Si/Al molar ratio of 10 or more and 300 or less with an alkaline earth metal compound is used and that when the Si/Al molar ratio of zeolite exceeds 300, effective acid sites of zeolite are reduced and the catalyst activity is lowered.

In addition, Patent Reference 3 describes a catalyst for obtaining propylene with high selectivity by inhibiting formation of aromatic compounds when propylene is manufactured from a reactor feed mixture (raw material mixture) containing methanol and/or dimethyl ether in the presence of an olefin having 4 or more carbon atoms.

### [Related Art References]

### [Patent References]

[Patent Reference 1] JP-T-2003-535069 (the term "JP-T" as used herein means a published Japanese translation of a PCT patent application)
[Patent Reference 2] JP-A-2008-080301
[Patent Reference 3] JP-A-2007-297363

### [Summary of the Invention]

### [Problems that the Invention is to Solve]

However, in the catalyst performance test of Patent Reference 2, a mixed gas of dimethyl ether and nitrogen is used as the reactor feed gas (raw material) and the examination is not made on the catalyst performance in a reactor feed gas composition (raw material composition) with the assumption of providing a reactor feed gas (raw material) to be used in the actual manufacture, namely a reactor feed gas (raw material) which contains various components that is not directly concerned in the reaction, illustratively olefins other than propylene formed by the reaction, as the reactor feed gas (raw material).

On the other hand, in the manufacture of propylene, it is advantageous in view of manufacture efficiency to construct an MTP process in which at least a part of olefins other than propylene contained in the gas formed by the reaction are recycled into the reactor with the aim of selectively manufacturing propylene.

Accordingly, great concern has been directed toward a technique for maintaining catalyst performance using recycled material, namely a catalyst for manufacturing propylene which can keep the catalyst performance at a high level even in a reaction system where an olefin having 4 or more carbon atoms is present.

However, it was revealed by the examination carried out by the present inventors that the catalyst disclosed in Patent Reference 2 has a problem of lowering catalyst performance in a reaction system where an olefin having 4 or more carbon atoms is present.

In addition, Patent Reference 3 discloses a zeolite as an effective active catalyst component, but it was revealed by the examination carried out by the present inventors that when the active catalyst component zeolite of Patent Reference 3 is actually used as the catalyst, it has a high selectivity but its catalyst performance is lowered.

Accordingly, when an application to actual manufacture is taken into consideration, it is necessary to use a shaped catalyst for the purpose of alleviating pressure drop of the catalyst bed, so that a shaped catalyst having further high durability is in demand.

By taking the above-mentioned problems into consideration, the object of the invention is to provide a catalyst for manufacturing propylene which can keep its stable activity for a prolonged period of time in manufacturing propylene by bringing an oxygen-containing compound into contact with the catalyst in the presence of an olefin having 4 or more carbon atoms, and a method for manufacturing propylene using said catalyst for manufacturing propylene.

### [Means for Solving the Problems]

With an aim of solving the above-mentioned problems, the present inventors have conducted intensive studies and found as a result that the optimum catalyst composition when an olefin having 4 or more carbon atoms is present in the reaction system is different from that when an olefin having 4 or more carbon atoms is not present in the reaction system.

Further, it was found that when an olefin having 4 or more carbon atoms is present, a silicalite having an Si/Al molar ratio of more than 300, namely markedly small Al quantity, shows sufficient catalyst activity. Under the condition of coexisting an olefin having 4 or more carbon atoms, when a silicalite having such a markedly small Al quantity is used as the catalyst, deactivation of the catalyst due to coke deposition is considerably inhibited.

In addition, it was found that, in making a composite by forming such a silicalite together with an alkaline earth metal compound, a catalyst which can be used stably for a more prolonged period of time can be obtained by making a composite containing at least one of an aluminum oxide and an aluminum hydroxide, in comparison with the case of using only silicon compound, such as a silicon oxide, a silicon hydroxide and a silicate, which does not contain at least one of an aluminum oxide and an aluminum hydroxide which is conventionally and generally used as a binder, thereby resulting in the accomplishment of the invention.

That is, the first aspect of the invention relates to a method for manufacturing propylene, which comprises, as a reaction step, bringing an oxygen-containing compound into contact with a catalyst in the presence of an olefin having 4 or more carbon atoms to manufacture propylene,
wherein the aforementioned catalyst is a composite comprising: a proton-type silicalite (to be referred to as a first component hereinafter); an alkaline earth metal compound (to be referred to as a second component hereinafter); and a non-zeolite compound comprising an aluminum oxide (to be referred to as a third component hereinafter), wherein
a molar ratio of Si/Al in the aforementioned first component is more than 300,
a content rate of the aforementioned second component to the aforementioned first component is 0.1% by mass or more in terms of alkaline earth metal, and
a content rate of the aforementioned third component in terms of Al2O3 to the aforementioned first component is 5% by mass or more and 50% by mass or less.

The second aspect of the invention relates to the above-mentioned method for manufacturing propylene, wherein the aforementioned first component is a silicalite comprising an MFI structure.

The third aspect of the invention relates to the above-mentioned method for manufacturing propylene, wherein the aforementioned second component is a calcium compound.

The forth aspect of the invention relates to the above-mentioned method for manufacturing propylene, wherein the aforementioned oxygen-containing compound is at least one of methanol and dimethyl ether.

The fifth aspect of the invention relates to the above-mentioned method for manufacturing propylene, which comprises bringing the aforementioned catalyst into contact with a steam atmosphere before the aforementioned reaction step.

The sixth aspect of the invention relates to the above-mentioned method for manufacturing propylene, which comprises bringing the aforementioned catalyst used in the aforementioned reaction step, to which a coke is adhered, into contact with an oxygen-containing gas to remove said coke to regenerate the catalyst, wherein the regenerated catalyst is supplied to the aforementioned reaction step.

The seventh aspect of the invention relates to the above-mentioned method for manufacturing propylene, which comprises bringing said regenerated catalyst into contact with a steam atmosphere before the regenerated catalyst is supplied to the aforementioned reaction step.

The eighth aspect of the invention relates to a catalyst for manufacturing propylene by bringing into contact with an oxygen-containing compound in the presence of an olefin having 4 or more carbon atoms,
wherein the aforementioned catalyst is a composite comprising: a proton-type silicalite (to be referred to as the first component hereinafter); an alkaline earth metal compound (to be referred to as the second component hereinafter); and a non-zeolite compound comprising an aluminum oxide (to be referred to as the third component hereinafter), wherein
a molar ratio of Si/Al in the aforementioned first component is more than 300,
a content rate of the aforementioned second component to the aforementioned first component is 0.1% by mass or more in terms of alkaline earth metal, and
a content rate of the aforementioned third component in terms of Al2O3 to the aforementioned first component is 5% by mass or more and 50% by mass or less.

The ninth aspect of the invention relates to the aforementioned catalyst for manufacturing propylene, wherein the aforementioned first component is a silicalite comprising an MFI structure.

The tenth aspect of the invention relates to the aforementioned catalyst for manufacturing propylene, wherein the aforementioned second component is a calcium compound.

### [Advantage of the Invention]

According to the catalyst of the invention for manufacturing propylene, in manufacturing propylene by bringing an oxygen-containing compound into contact with the catalyst in the presence of an olefin having 4 or more carbon atoms, propylene can be manufactured stably and efficiently for a prolonged period of time by inhibiting deactivation of the catalyst.

In addition, according to the catalyst of the invention for manufacturing propylene, an MTP process for selectively manufacturing propylene can be effectively constructed by recycling the formed olefins other than propylene into the reactor in MTO process, which is industrially markedly advantageous.

### [Mode for Carrying Out the Invention]

The following illustratively describes typical embodiments for carrying out the invention, though the invention is not limited to the following embodiments.

### [Catalyst for manufacturing propylene]

Firstly, the catalyst of the invention for manufacturing propylene is described.

The catalyst of the invention for manufacturing propylene is a composite comprising: a proton-type silicalite (a first component); an alkaline earth metal compound (a second component); and a non-zeolite compound comprising an aluminum oxide
(a third component), wherein a molar ratio of Si/Al in the aforementioned first component is more than 300, a content rate of the aforementioned second component to the aforementioned first component is 0.1% by mass or more in terms of alkaline earth metal, and a content rate of the aforementioned third component in terms of Al2O3 to the aforementioned first component is 5% by mass or more and 50% by mass or less.

In this connection, the term "composite" as used herein means a solid of a mixture comprising the above-mentioned first component, second component and third component.

The following describes each requirement of the catalyst of the invention in detail.

### <First component>

The first component of the catalyst according to the invention is a proton-type silicalite (hereinafter, these may be generally called simply "silicalite").

In this connection, the silicalite generally represents a zeolite which does not contain Al at all or has an extremely small amount of Al, and the first component concerned in the invention is defined as "silicalite" because the Si/Al molar ratio is more than 300 and the Al content is extremely small as described in the above.

The Si/Al molar ratio of the aforementioned first component is more than 300, preferably 350 or more. In addition, in general, this is preferably 1,000 or less, more preferably 800 or less.

When the Si/Al molar ratio is less than the above-mentioned lower limit, deposition of coke is accelerated so that the catalyst life becomes short. Also, by setting the Si/Al molar ratio at the aforementioned upper limit or less, sufficient catalytic activity can be obtained because reduction of effective acid sites of silicalite, namely active sites for the reaction, is prevented. This can prevent the amount of catalyst for the reaction from increasing.

Though the method for adjusting the Si/Al molar ratio of the first component is not particularly limited, there are a method in which material ratio at the time of catalyst manufacture is adjusted, and a method in which the manufactured zeolite is dealuminated with high temperature steam, and the like, but in general, it is adjusted by the material ratio at the time of catalyst manufacture.

Thus, according to the invention, silicalite which has larger Si/Al molar ratio than that of the catalyst used in Patent Reference 2 is effective in preventing deactivation of catalyst, due to the following reasons.

That is, like the case of Patent Reference 2, for example, when at least one of methanol and dimethyl ether is supplied as the oxygen-containing compound of the reaction raw material, conversion of the at least one of methanol and dimethyl ether is slow so that sufficient activity cannot be obtained by a zeolite having an Si/Al molar ratio of more than 300.

However, the present inventors have found that when an olefin having 4 or more carbon atoms is coexisted, conversion of the oxygen-containing compound is accelerated by said olefin and the conversion therefore is markedly improved. Accordingly, sufficient reaction activity can be obtained even by the silicalite having extremely small amount of Al, and on the other hand, deposition of coke is suppressed due to the small amount of Al so that deactivation of the catalyst is inhibited.

As the structure of the silicalite according to the invention, there is no particular limitation, but for example when represented by the codes defined by International Zeolite Association (IZA), AEI, AET, AEL, AFI, AFO, AFS, AST, ATN, BEA, CAN, CHA, DDR, EMT, ERI, EUO, FAU, FER, LEV, LTL, MAZ, MEL, MFI, MOR, MTT, MTW, MWW, OFF, PAU, RHO, STT, TON and the like can be used.

Particularly, MFI, MEL, MOR and MWW are desirable, and MFI is more desirable. These may be used alone or as a mixture of two or more.

The pore diameter of the silicalite according to the invention is not particularly limited, but in general, 0.3 nm or more is desirable, and 0.9 nm or less is more desirable. In this connection, the term "pore diameter" as used herein means a crystallographic channel diameter (crystallographic free diameter of the channels) defined by International Zeolite Association (IZA). When shape of the pore (channel) is completely round, it indicates diameter, and when shape of the pore is oval, it indicates minor axis.

In addition, average particle diameter of the silicalite according to the invention is not particularly limited, but in general, 0.001 µm or more is desirable, and 0.005 µm or more is more desirable. Also in general, 20 µm or less is desirable, and 10 µm or less is more desirable.

By setting average particle diameter of silicalite to the aforementioned upper limit or less, lowering of the surface area which shows the catalytic activity can be prevented and lowering of the activity can also be prevented. Also, by setting it to the aforementioned lower limit or more, those which have excellent handling ability can be obtained.

The average particle diameter of the silicalite according to the invention can be calculated by a laser diffraction measurement. However, since 0.1 µm or less cannot be measured by the laser diffraction method, when the result measured by the laser diffraction method is 0.1 µm or less, the value can be known by a scanning electron microscopy (SEM) analysis is used as the average particle diameter.

The preparation method of silicalite according to the invention is not particularly limited, and it is possible to prepare it by a conventionally known method such as the method described in the Journal of The Japan Petroleum Institute, 24 (5), 275 to 280 (1981). In general, it is prepared by the following hydrothermal synthesis.

That is, in general, silicalite is obtained by mixing a silicon atom (Si) source, an aluminum atom (Al) source, water and, in response to the necessity, at least one of an alkali and a structure directing agent (template), and then carrying out hydrothermal synthesis by allowing them to undergo the reaction under a high temperature high pressure condition, subsequently removing the template in the case that the template was added.

As the aforementioned silicon atom source, illustratively, for example, sodium silicate, colloidal silica, fumed silica, silica sol and the like can be mentioned.

As the aforementioned aluminum atom source, illustratively, for example, aluminum hydroxide, sodium aluminate, alumina sol, pseudo-boehmite, aluminum alkoxides such as aluminum isopropoxide and aluminum triethoxide, and the like can be mentioned.

As the aforementioned alkali, illustratively, for example, sodium hydroxide, sodium aluminate, sodium silicate, potassium hydroxide and the like can be mentioned.

As the aforementioned structure directing agent, illustratively, for example, a quaternary ammonium salt, a primary amine, a secondary amine, a tertiary amine, polyamine and the like can be mentioned. These may be used as a mixture.

An aqueous gel is prepared by mixing the above-mentioned silicon atom source, aluminum atom source, water and, in response to the necessity, at least one of the alkali and structure directing agent. The mixing order is not particularly limited, but in general, the silicon atom source and aluminum atom source are firstly mixed and at least one of the alkali and structure directing agent is mixed therewith.

### (Hydrothermal synthesis)

Hydrothermal synthesis of silicalite is carried out by putting the aqueous gel into an autoclave and a predetermined temperature is maintained under a self-generating pressure or under pressurization of a gas which does not inhibit crystallization, with or without stirring.

As the conditions of hydrothermal synthesis, from 100 to 300°C is generally desirable, and from the viewpoint of easiness of synthesis, from 120 to 250°C is more desirable and from 140 to 200°C is particularly desirable. As the reaction time, from 1 hour to 30 days is generally desirable, and from the viewpoint of easiness of synthesis, from 2 hours to 15 days is more desirable and from 4 hours to 2 days is particularly desirable.

After the hydrothermal synthesis, the product is separated, washed with water and dried, and the contained organic compound is removed partly or entirely by a method such as a calcination which uses air or the like, thereby obtaining the silicalite concerned in the invention.

It is possible to change composition of the silicalite after hydrothermal synthesis by modifications such as ion exchange, dealumination, impregnation and supporting.

The silicalite to be used in the invention may be any substance with the proviso that it has the aforementioned composition when subjected to the reaction, and may be a substance prepared by any method.

### <Second component>

The second component of the catalyst concerned in the invention is an alkaline earth metal compound. By the addition of such a second component, coke deposition and dealumination of the silicalite as the aforementioned first component are inhibited, so that deactivation of the catalyst can be prevented.

Though the reason why the second component inhibits deterioration of the catalyst is not clear, it is considered that it is due to the control of acid strength of silicalite within an appropriate range by the interaction of alkaline earth metal compound and acid sites of silicalite .

The alkaline earth metal compound as the second component is not particularly limited, and for example, carbonates, hydroxides, oxides, acetates, nitrates, aluminates, ortho-silicates and the like of alkaline earth metals can be used. Particularly, carbonates and acetates of alkaline earth metals are desirable.

Kinds of the alkaline earth metal are not particularly limited, and for example, calcium, magnesium and strontium are used. Particularly, a calcium compound is desirable because it can be used inexpensively.

That is, calcium carbonate and calcium acetate are desirable as the second component. These second components may be used alone or as a mixture of two or more species.

A content rate of the second component to the first component in the catalyst of the invention is 0.1% by mass or more, preferably 0.3% by mass or more, more preferably 1.0% by mass or more, further more preferably 2.0% by mass or more, in terms of alkaline earth metal. Also, generally 20% by mass or less is preferable and 10% by mass or less is preferable.

By setting the content rate of the second component to the above-mentioned lower limit or more, it is desirable because the coke deposition is inhibited and the effect to inhibit dealumination of the silicalite as the first component also becomes sufficient. On the other hand, side reactions can be inhibited by setting the content rate of the second component to the above-mentioned upper limit or less.

In addition, the average particle diameter of the alkaline earth metal compound according to the present invention is not particularly limited, but is generally preferably 0.001 µm or more, particularly preferably 0.005 µm or more, and generally preferably 20 µm or less, particularly preferably 10 µm or less.

By setting the average particle diameter to the above-mentioned upper limit or less, the diffusion of reactants in zeolite crystals can be easier, which leads to the suppression of coke deposition and dealumination. In addition, by setting the average particle diameter to the above-mentioned lower limit or more, it can make handling ability easy.

The average particle diameter of the alkaline earth metal compound can be determined by a laser diffraction method or a scanning electron microscopy (SEM) analysis.

### <Third component>

The third component of the catalyst concerned in the invention is a non-zeolite compound comprising an aluminum oxide ,
namely a compound other than zeolite, as defined in the claims.

In this case, as the aluminum oxide,
for example, γ-alumina, boehmite, alumina sol and the like can be mentioned. These may be used alone or as a mixture of two or more species.

As the third component, it may be any substance which comprises
an aluminum oxide as mentioned above, and it may also comprise a component other than an aluminum oxide, for example, additionally
an aluminum hydroxide.

As the component other than an aluminum oxide
to be contained as the third component, for example, an oxide of silicon, a hydroxide of silicon and clay such as kaolin and bentonite can be mentioned. These may be used alone or as a mixture of two or more species.

The main role of the third component of the catalyst of the invention is a role as a binder for binding the silicalites each other as the first component in order to keep strength of the catalyst. However, the third component may have not only the function as a binder, but may exert influence upon the catalyst life in some cases.

For example, when at least one of a silicon oxide and silicon hydroxide is used as the third component, it is not desirable because though it is possible to keep strength of the catalyst, long-term life of the catalyst (accumulated reaction time when the reaction is carried out by repeating regeneration of the catalyst) becomes short in comparison with the case of using an aluminum oxide.

Though the reason for this is not clear, when an aluminum oxide is used, it is considered a possibility that the aluminum contained in an aluminum oxide is reintroduced into the sites where aluminum is eliminated of silicalite.

A content rate of the third component to the first component in the catalyst described herein is 5% by mass or more and 200% by mass or less, preferably 10% by mass or more, more preferably 15% by mass or more, particularly preferably 25% by mass or more. Also, 100% by mass or less is preferable and 50% by mass or less is more preferable. In the claimed invention, a content rate of the third component in terms of Al2O3 to the first component in the catalyst is 5% to 50% by mass.

By setting the content rate of the third component to the first component to the above-mentioned lower limit or more, the strength of the obtained catalyst becomes sufficient, and to cause problems such as partial powderization when used can be inhibited. On the other hand, by setting the content rate of the third component to the first component to the above-mentioned upper limit or less, lowering the activity per catalyst mass caused by relatively reducing the ratio of the first component which shows activity for the reaction can be inhibited.

In this connection, as described in the foregoing, the third component in the catalyst of the invention may be a substance consisting of an aluminum oxide and optionally an aluminum hydroxide or may be a substance consisting of an aluminum oxide , optionally an aluminum hydroxide and a component other than the aluminum oxide and the optional aluminum hydroxide, but it is desirable to adjust the amount of the aluminum oxide and the optional aluminum hydroxide in the third component by setting the rate of the third component to the first component to the above-mentioned range. By this, the effect of the invention by the use of the third component comprising an aluminum oxide and optionally an aluminum hydroxide can be obtained further more definitely.

It is desirable to adjust the amount of an aluminum oxide and an optional aluminum hydroxide in the third component in such a manner that the content rate in terms of Al in the third component becomes preferably 1% by mass or more, more preferably 5% by mass or more, based on the first component. Also, it is desirable to adjust in such a manner that it becomes preferably 100% by mass or less, more preferably 50% by mass or less.

### <Other component>

In addition, the catalyst of the invention may contain a component other than the above-mentioned first component to third component in response to the necessity. As the other component, a component other than those which have a function as a binder, which is different from the above-mentioned third component, such as a component having a function to improve productivity at the time of manufacturing the catalyst can be mentioned.

As the other component, illustratively, for example, graphite, celluloses, other additives and the like can be mentioned. These may be used alone or as a mixture of two or more species.

### <Alkali metal>

There is a case in which an alkali metal is contained in the catalyst of the invention when the catalyst is manufactured using a material containing the alkali metal. The amount of the alkali metal compounds contained in the catalyst of the invention is preferably 0.1% by mass or less, more preferably 0.06% by mass or less, further preferably 0.03% by mass or less, in terms of alkali metals.

By setting the amount of the alkali metal compounds to the above-mentioned upper limit or less, lowering of the catalyst activity due to interaction with the acid sites of silicalite can be inhibited.

### <Shape>

The shape of the catalyst of the invention is not particularly limited, but for example, those which are extruded into a pellet type, a cylindrical type, a ring shape, a trefoil cross section type, a quatrefoil cross section type, a wheel type extrusion, and formed into a spherical shape are used.

### <Size>

The size of the catalyst of the invention is not particularly limited either, but in general, it is desirable that the thickness is 0.1 mm or more and 2 mm or less, where the thickness is defined as a distance twice the maximum value among the shortest distances from arbitrary points inside the molded catalyst to the outer surface.

Setting of the thickness of the catalyst to 0.1 mm or more renders possible prevention of lowering of the propylene yield due to enlarged pressure drop of the catalyst bed at the time of subjecting to the reaction. On the other hand, setting of the thickness of the catalyst to 2 mm or less renders possible prevention of lowering of the propylene yield per catalyst mass due to insufficient diffusion of the reaction raw material into inside of the catalyst.

### <Preparation method of catalyst>

Preparation method of the catalyst of the invention is described in the following. Firstly, the aforementioned first component, second component and third component and a polar solvent, or the aforementioned other components in response to the necessity, are mixed and kneaded using a mortar, a Raikai mixer, a kneader or the like, thereby preparing a mixture containing the first component, second component, third component and polar solvent (mixing and kneading step).

In this mixing and kneading step, blending ratio of respective components is adjusted in such a manner that a content rate of the second component to the first component becomes 0.1% by mass or more in terms of alkaline earth metal, and a content rate of the third component in terms of Al2O3 to the first component becomes 5% by mass or more and 200% by mass or less, in the claimed invention 5% to 50% by mass.

The adding amount of the polar solvent is not particularly limited, but in general, 10% by mass or more is desirable and 150% by mass or less is desirable, based on the total mass of the first component, second component and third component.

As the polar solvent, water is most suitable, but for example, alcohols such as methanol, ethanol and propanol, ethers such as diethyl ether and tetrahydrofuran, other esters, nitriles, amides, sulfoxides and the like can also be used. In addition, these can also be used in a mixed state.

Also, in addition to the polar solvent, for example, the additives such as organic acids such as acetic acid, amine compounds such as ammonia, graphite, celluloses and the like, which are removed in the drying and calcination step of the subsequent stage, may be added.

Next, a molded product is obtained by molding the mixture obtained by the mixing and kneading step, by an extrusion molding which uses an extruder, a spherical body molding by Marumerizer, and the like (molding step).

Next, the molded product obtained by the molding step is dried by a dryer and then calcined using furnace such as muffle furnace or tunnel furnace (drying and calcination step) to obtain the catalyst of the invention.

As drying conditions of the molded product in this drying and calcination step, the drying temperature is preferably 80°C or more and preferably 150°C or less. Also, the drying period of time is preferably 0.5 hour or more, and for example, it is desirable to dry it for a period of from 5 to 24 hours.

Setting of the drying temperature or drying time to the aforementioned lower limit or more renders possible prevention of the polar solvent from remaining inside the molded product and from becoming a cause of structural breaking of silicalite in the calcination step. In addition, its setting to the aforementioned upper limit or less renders possible prevention of the polar solvent from becoming a cause of structural breaking of silicalite at the time of drying due to exposure of silicalite to high temperature under such a state that the polar solvent is present inside the molded product.

In addition, regarding calcination conditions of the molded product after drying in this drying and calcination step, the calcination temperature is preferably 350°C or more and preferably 750°C or less. Also, the time of calcination is preferably 1 hour or more, and for example, it is desirable to carry out the calcination for a period of from 2 to 24 hours.

By setting the calcination temperature or calcination time to the aforementioned lower limit or more, catalyst strength can be sufficiently obtained. Also, by setting the calcination temperature or calcination time to the aforementioned upper limit or less, the structure disorder of silicalite can be prevented.

### [Method for manufacturing propylene]

The following describes the method for manufacturing propylene of the invention which uses the catalyst of the invention for manufacturing propylene described in the above.

In the method for manufacturing propylene of the invention, propylene is manufactured by bringing an oxygen-containing compound into contact with the above-mentioned catalyst for manufacturing propylene in the presence of an olefin having 4 or more carbon atoms.

### <Reaction raw material>

Regarding the oxygen-containing compound as the raw material of the reaction, for example, there may be mentioned one or two or more species of alcohols, ethers, ketones, carboxylic acids and the like. Among them, alcohols and ethers are desirable, at least one of an alcohol having from 1 to 3 carbon atoms and an ether having from 2 to 6 carbon atoms is more desirable, and at least one of methanol and dimethyl ether is particularly desirable.

As an example of an embodiment of the method for manufacturing propylene of the invention, a case in which at least one of methanol and dimethyl ether is used as the oxygen-containing compound is exemplified and described in the following, though the oxygen-containing compound concerned in the invention is not limited to the at least one of methanol and dimethyl ether.

Manufacture origin of the at least one of methanol and dimethyl ether to be used as the reaction raw material is not particularly limited. For example, there may be mentioned coal and natural gas, those which are obtained by hydrogenation reaction of hydrogen/CO mixed gas derived from a by-product in the steel industry, those which are obtained from plants, those which are obtained from organic substances such as recycled plastics and municipal waste, and the like. In this case, a material in which compounds other than methanol and dimethyl ether originated from respective manufacture method are optionally mixed may be used as such, or a purified material thereof may be used.

According to the invention, the olefin having 4 or more carbon atoms to be coexisted in bringing an oxygen-containing compound into contact with the catalyst is preferably an olefin having 4 or more and 10 or less carbon atoms, more preferably an olefin having 4 or 5 carbon atoms. These may be used alone or as a mixture of two or more species. It may be either a straight chain olefin or a branched olefin or may be a mixture thereof.

Since the olefins having 4 or more carbon atoms are generally obtained as by-products of the propylene manufacture in the invention, the olefins having 4 or more carbon atoms remained after separation of the propylene as the intended substance from the reaction products can be coexisted with an oxygen-containing compound by recycling into the reaction step in the separation step of the subsequent stage of the reaction step.

Also, in addition to such olefins having 4 or more carbon atoms as by-products, an olefin having 4 or more carbon atoms may be newly supplied as a reaction material. The olefin having 4 or more carbon atoms as the reaction raw material is not particularly limited.

For example, an olefin having 4 or more, particularly from 4 to 10, carbon atoms obtained by conventionally known various methods can be optionally used, such as those which are produced from a petroleum supply material by a catalytic cracking method or a steam cracking method (C4 raffinate-1 and C4 raffinate-2, and the like), those which are obtained by carrying out FT (Fischer-Tropsch) synthesis using, as the material, a hydrogen/CO mixed gas obtained by the gasification of coal, those which are obtained by an oligomerization reaction of ethylene including dimerization reaction, those which are obtained by a dehydrogenation method or oxidation dehydrogenation method of a paraffin having 4 or more carbon atoms, those which are obtained by an MTO reaction, those which are obtained by a dehydration reaction of an alcohol, those which are obtained by a hydrogenation reaction of a diene compound having 4 or more carbon atoms, and the like.

A material under a state of being optionally mixed with a compound other than the olefin having 4 or more carbon atoms originated from respective method may be used as such, or a purified olefin thereof may be used.

### <Steam treatment of catalyst>

The catalyst obtained in the aforementioned drying and calcination step can manufacture high propylene yield even when brought into contact with an oxygen-containing compound as it is, and also, it is possible to use it stably for a prolonged period of time because deposition of coke is small. However, when said catalyst is subjected to the reaction after carrying out a pretreatment under a steam atmosphere (steam treatment step), it becomes possible to use it stable for a prolonged period of time because deposition of coke is further inhibited, which is desirable.

Though the reason for this is not clear, it is considered that acid sites originating from an aluminum oxide and optionally aluminum hydroxide added as the third component are reduced, or acid strength of these acid sites is lowered, by the steam treatment, so that the coke deposition which uses said acid sites as the starting point is inhibited.

Partial pressure of steam in the steam treatment step is not particularly limited, but 0.01 MPa (absolute pressure, the same shall apply hereinafter) or more is generally preferable and 0.03 MPa or more is further preferable. Also, 0.50 MPa or less is generally preferable and 0.30 MPa or less is further preferable.

When the steam partial pressure in the steam treatment is set to the aforementioned lower limit or more, the effect to inhibit deposition of the coke can be obtained sufficiently. On the other hand, when the steam partial pressure is set to the aforementioned upper limit or less, dealumination of silicalite can be prevented.

In the steam treatment step, steam alone may be used, but the steam may be diluted with at least one of air and inert gases (nitrogen, carbon dioxide and the like). In addition, the catalyst may be brought into contact with a reaction atmosphere which forms steam.

Temperature of the steam treatment step is not particularly limited, but 300°C or more is generally preferable, 350°C or more is more preferable and 400°C or more is particularly preferable. Also, 700°C or less is generally preferable, 650°C or less is more preferable and 600°C or less is particularly preferable.

When the temperature in the steam treatment is set to the aforementioned lower limit or more, the effect to inhibit deposition of the coke can be obtained sufficiently. On the other hand, when the temperature is set to the aforementioned upper limit or less, dealumination of silicalite can be prevented.

The treating period of time in the steam treatment step is not particularly limited, but 1 hour or more is generally desirable and 5 hours or more is further desirable. Also, 100 hours or less is generally desirable and 50 hours or less is further desirable.

### <Reaction operation and condition>

Operations and conditions of the propylene manufacture reaction of the invention which uses the aforementioned catalyst and reaction materials are described in the following.

### (Reactor)

The reaction for manufacturing propylene from an oxygen-containing compound in the invention is a gas phase reaction. The mode of this gas phase reactor is not particularly limited, but for example, a continuous type fixed bed reactor, a moving bed reactor and a fluidized bed reactor can be mentioned. Among them, a fixed bed reactor and a moving bed reactor are preferable and a fixed bed reactor is particularly preferable.

When a moving bed reactor or a fluidized bed reactor is used, a complex reactor designing is required and at the same time, the shape and strength of the catalyst are limited, but in the case of the employment of a fixed bed reactor, it has a characteristic in that the reactor designing and catalyst designing can be made relatively easily.

In this connection, in case that the catalyst life is extremely short, it becomes necessary to employ a moving bed reactor or fluidized bed reactor which can continuously regenerate the catalyst while moving it because of the necessity to carry out regeneration of catalyst after carrying out a short period of time of the reaction, but since the catalyst of the invention can be subjected to the reaction stably for a prolonged period of time due to less deposition of coke and long catalyst life, employment of a fixed bed reactor is desirable.

As the mode of the fixed bed reactor, for example, a multitubular reactor and a adiabatic reactor can be mentioned.

In this connection, in order to keep temperature distribution of the catalyst bed small in packing the aforementioned catalyst in a reactor, reaction inert particulate matter such as quartz sand, alumina, silica and the like may be mixed with the catalyst and packed. In that case, there is no particular limitation on the using amount of the reaction inert particulate matter such as quartz sand. In this connection, it is desirable that said particulate matter has a particle diameter similar to that of the catalyst in view of the uniform mixing ability with the catalyst.

### (Temperature and Pressure)

The method for manufacturing propylene of the invention can be carried out under broad range of temperature and pressure conditions.

Regarding the reaction temperature, it is generally 400°C or more, desirably 450°C or more, and more desirably 500°C or more. Also, it is generally 700°C or less, desirably 600°C or less, and more desirably 580°C or less. The term "reaction temperature" as used herein means temperature of the gas formed by the reaction at the catalyst bed outlet in the reactor.

When the reaction temperature is set to the aforementioned lower limit or more, sufficient catalyst activity can be obtained. Also, when the reaction temperature is set to the aforementioned upper limit or less, dealumination of silicalite and increase of the deposition rate of the coke can be inhibited.

The reaction pressure is not particularly limited, 1 MPa (absolute pressure, the same shall apply hereinafter) or less is generally desirable, 0.5 MPa or less is more desirable and 0.3 MPa or less is particularly desirable. Also, 1 kPa or more is generally desirable, 50 kPa or more is more desirable and 100 kPa or more is particularly desirable. The term "reaction pressure" as used herein means an average value obtained by adding the pressure at the catalyst bed inlet and the outlet pressure, and dividing the result by 2.

When the reaction pressure is set to the aforementioned upper limit or less, lowering of the propylene yield can be prevented and at the same time, increase of the deposition rate of the coke can be prevented. In addition, when it is set to the aforementioned lower limit or more, sufficient catalyst activity can be obtained.

### (Feed ratio and feed amount of raw materials)

The amount of the olefin having 4 or more carbon atoms to be fed to the reactor is not particularly limited, 0.1 by molar ratio is generally desirable and 0.2 or more is more desirable, based on the total of the mol number of methanol and 2 times of the mol number of dimethyl ether to be supplied to the reactor. Also, as the molar ratio, 10 or less is generally desirable and 5 or less is more desirable. When this feed molar ratio is set to the aforementioned lower limit or more and upper limit or less, lowering of the reaction rate can be prevented.

In addition, regarding the total concentration of the olefin having 4 or more carbon atoms, methanol and dimethyl ether (substrate concentration) among all of the components to be fed to the reactor, there is no particular limitation, but 90% by mol or less is generally desirable and 80% by mol or less is desirable. Also, 20% by mol or more is generally desirable.

When the aforementioned substrate concentration is set to the aforementioned upper limit or less, lowering of the propylene yield can be prevented and at the same time, increase of the deposition rate of the coke can be prevented. When the substrate concentration is less than the aforementioned lower limit, there may be a case in which the catalyst activity becomes insufficient. Accordingly, it is desirable to dilute the reaction materials with the dilution gas described in the following in response to the necessity so that such a substrate concentration can be obtained.

Regarding feeding method of the raw material to the reactor, a mixture of the oxygen-containing compound and olefin having 4 or more carbon atoms may be supplied or these may be fed separately. When a material is liquid, a gas fluid vaporized using an vaporizer is used as the material.

In addition, when the oxygen-containing compound is methanol, a gas prepared by bringing at least a part of vaporized methanol into contact with a solid acid catalyst and thereby converting into dimethyl ether and water may be fed to the reactor.

### (Dilution gas)

As the dilution gas, a gas inert to the reaction or having extremely low reactivity is desirable. As said gas, for example, there may be mentioned paraffins, steam, nitrogen, helium, argon, carbon monoxide, carbon dioxide, hydrogen and a mixture of two or more of these, and the like. Among these, paraffins and steam are desirable.

As the aforementioned dilution gas, those which are contained in the reaction materials may be used as such, or those which are by-produced by the reaction of the invention may be separated and then used by recycling them. In addition, a separately prepared dilution gas may be used.

### (Space velocity)

Weight hourly space velocity (to be referred to as "WHSV" hereinafter) of the gas to be fed to the reactor is not particularly limited, but as an olefin-based WHSV, 0.5 Hr⁻¹ or more is generally desirable and 1.0 Hr⁻¹ or more is more desirable. Also, 10 Hr⁻¹ or less is generally desirable and 5.0 Hr⁻¹ or less is more desirable.

The "WHSV" as used herein indicates total flow rate (weight/hour) of the methanol, dimethyl ether and olefin having 4 or more carbon atoms to be fed per catalyst unit weight, and the olefin-based WHSV is a value based on the weight of the methanol and dimethyl ether under a dehydrated state.

That is, in the case of methanol, it is considered as CH₃OH → CH₂ + H₂O, and CH₂ alone is calculated as the weight. Illustratively, "flow rate of methanol × 14 (molecular weight of CH₂)/32 (molecular weight of methanol)" is used as the olefin-based methanol flow rate.

Also, in the case of dimethyl ether, in the same manner, it is considered as CH₃OCH₃ → C₂H₄ + H₂O, and "flow rate of dimethyl ether × 28 (molecular weight of C₂H₄)/46 (molecular weight of dimethyl ether)" is used as the olefin-based dimethyl ether flow rate.

When this olefin-based WHSV is less than the aforementioned lower limit, the productivity per reactor unit volume tends to become low and the propylene yield also tends to be lowered. When the olefin-based WHSV exceeds the aforementioned upper limit, the propylene yield tends to be lowered and the catalyst life also tends to be shortened.

### (Reactor outlet gas)

According to the invention, a mixed gas comprising propylene as the specified substance and, as by-products, olefins other than propylene such as ethylene, butane and pentene, paraffins, aromatic compounds, water and the like is obtained as the reactor outlet gas. It is desirable that the concentration of propylene in said mixed gas is adjusted to generally from about 5 to about 80% by weight.

### <Catalyst regeneration step>

According to the method for manufacturing propylene of the invention, deposition of coke is small in comparison with the conventional catalysts and the rate of lowering of the catalyst activity is slow, but in the case of carrying out one year or more of the continuous operation, it is desirable to include a step of regenerating the catalyst by removing the coke (catalyst regeneration step) during the operation.

For example, when a fixed bed reactor is selected, it is desirable to arrange at least two or more reactors and to carry out the operation by alternately switching the reaction and the catalyst regeneration.

On the other hand, when a moving bed reactor or fluidized bed reactor is selected, it is desirable to carry out the reaction by continuously transferring the catalyst to a regenerator and returning the catalyst regenerated in the regenerator continuously into the reactor.

A method in the case of employing a fixed bed reactor is described in the following as an example of an embodiment of the catalyst regeneration step.

As the gas to be contacted with the catalyst for regenerating the catalyst, there is no particular limitation, but for example, oxygen, hydrogen, a gas including steam, a mixture thereof and the like can be mentioned. Particularly, regeneration by an oxygen-containing gas is desirable. That is, it is desirable to carry out regeneration of the catalyst by supplying an oxygen-containing gas into a fixed bed reactor where regeneration of the catalyst is carried out.

The concentration of oxygen at the catalyst bed inlet of the fixed bed reactor in the catalyst regeneration step by an oxygen-containing gas is generally preferably 0.1% by mol or more and more preferably 0.5% by mol or more. Also, 21% by mol or less is generally preferable, 5% by mol or less is more preferable and 2% by mol or less is particularly preferable.

Accordingly, it is desirable to dilute the gas to be used in the regeneration of the catalyst with the dilution gas described in the following, in response to the necessity so that the aforementioned oxygen concentration is obtained.

As the dilution gas, a gas inert to the catalyst is desirable. As said gas, for example, steam, nitrogen, helium, argon, carbon monoxide, carbon dioxide, hydrogen, a mixture of two or more species thereof and the like can be mentioned. Among them, nitrogen is desirable.

The pressure in the catalyst regeneration step is not particularly limited, but generally 1 MPa (absolute pressure, the same shall apply hereinafter) or less is desirable and 0.5 MPa or less is more desirable. Also, generally 1 kPa or more is desirable, 50 kPa or more is more desirable and 100 kPa or more is further desirable. The term "pressure" as used herein means an average value obtained by adding the pressure at the catalyst bed inlet of the fixed bed reactor and the outlet pressure, and dividing the result by 2.

Though the temperature of the catalyst regeneration step is not particularly limited, generally 400°C or more is desirable, 450°C or more is more desirable and 500°C or more is particularly desirable. Also, generally 700°C or less is desirable, 600°C or less is more desirable and 580°C or less is particularly desirable.

The aforementioned temperature is the temperature of catalyst regeneration gas at the catalyst bed outlet in the reactor. When said temperature is adjusted at the aforementioned lower limit or more, the coke removing effect can be sufficiently obtained. Also, when said temperature is adjusted at the aforementioned upper limit or less, structural breaking of silicalite can be inhibited.

Though the treating period of time in the catalyst regeneration step is not particularly limited, it is generally 1 hour or more and preferably 5 hours or more, and generally 100 hours or less and preferably 50 hours or less.

Though the flow rate of the regeneration gas per catalyst unit weight (Nm³/Hr-kg-catalyst) in the catalyst regeneration step is not particularly limited, generally from 0.5 to 50 Nm³/hr·kg-catalyst is desirable and from 1 to 15 Nm³/hr·kg-catalyst is more desirable.

In the catalyst regeneration step by an oxygen-containing gas, a mixed gas comprising carbon dioxide, carbon monoxide, water, unreacted oxygen and the like is obtained as the reactor outlet gas. Entire portion of these gases may be discarded in accordance with a conventionally known method, but it is desirable to recycle and reuse at least a part thereof as a regenerated gas. By this, it is possible to reduce amount of the gas to be used newly.

The catalyst after regeneration can be used in the manufacture of propylene by bringing it into contact with the material including an oxygen-containing compound in the presence of an olefin having 4 or more carbon atoms again, and in that case, it is desirable to bring the catalyst after regeneration into contact with steam under the steam treatment condition described in the <Steam treatment of catalyst> and then bring the catalyst into contact with the material. By this, effects similar to the effects described in the <Steam treatment of catalyst> can be obtained. The regeneration of the above catalyst can be repeatedly performed, and the activity can be maintained even if the regeneration of the catalyst of the present invention is repeatedly performed preferably three times or more, and more preferably ten times or more.

### <Separation of products>

The mixed gas comprising propylene and by-products and the like, as the reactor outlet gas, may be introduced into conventionally known separation purification facilities to carry out recovery, purification, recycle and discharge treatments in response to respective components.

It is desirable that a part or entire portion of the components other than propylene is recycled after the above-mentioned separation and purification by mixing with the reaction material or supplying directly to the reactor.

Particularly, when the fluid containing an olefin having 4 or more carbon atoms is recycled into the reactor, conversion of an oxygen-containing compound as the material can be increased and the reaction can be carried out under conditions suitable for the use of the catalyst of the invention, which are desirable.

### [Examples]

The following describes the invention more illustratively based on examples, though the invention is not limited to the following examples.

### [Preparation of catalyst]

### <Preparation Example 1>

By preparing an aqueous gel in accordance with the method described in Journal of The Japan Petroleum Institute, 24 (5), 275 to 280 (1981), hydrothermal synthesis was carried out in an autoclave. The product was subjected to pressure filtration, and the solid component was separated, thoroughly washed with water and then dried at 100°C for 24 hours. The solid after drying was calcined at 550°C for 6 hours under a flow of air.

The calcined product was suspended in 1 M ammonium nitrate aqueous solution and stirred at 80°C for 2 hours. Thereafter, the solid component was separated by filtration at room temperature, thoroughly washed with water, again suspended in 1 M ammonium nitrate aqueous solution and stirred at 80°C for 2 hours. Thereafter, the solid component was separated by filtration at room temperature, thoroughly washed with water and then dried at 100°C for 24 hours to obtain an ammonium-type silicalite powder.

It was confirmed by XRD that this ammonium-type silicalite powder had an MFI structure. In addition, as a result of elemental analysis, the Si/Al molar ratio was 450 and average particle diameter was 4 µm.

A mixture was prepared by mixing 100 g of the above-mentioned ammonium-type silicalite powder with 36 g of boehmite (70% by mass in Al₂O₃-equivalent amount) and 5.0 g of calcium carbonate (average particle diameter: 5 µm) and further kneading by adding an appropriate amount of ion exchange water. This mixture was subjected to extrusion molding using an extruder having a dice diameter of 1.8 mm, and the resulting molded product was dried at 120°C for 5 hours, followed by calcination at 550°C for 10 hours, thereby obtaining an extruded cylindrical catalyst of 1.8 mm in diameter and 5 mm in length. This catalyst was named catalyst A.

In the catalyst A, the Si/Al molar ratio of the first component ammonium-type silicalite was 450. Also, the content rate of the calcium carbonate as the second component to the first component was 2.0% by mass in terms of alkaline earth metal, and the content rate of Al₂O₃ as the third component to the first component was 25% by mass. In addition, the amount of the alkali metal compound in terms of alkali metal was 0.018% by mass.

### <Preparation Example 2>

100 g of the ammonium-type silicalite powder of Preparation Example 1 was mixed with 5.0 g of calcium carbonate and an appropriate amount of methyl cellulose as a molding additive, and then 20 g of methyl silicate (MS56S, mfd. by Mitsubishi Chemical Corp.) was added thereto. A mixture was prepared by kneading after adding an appropriate amount of ion exchange water. This mixture was molded, dried and calcined in the same manner as in Preparation Example 1 to obtain an extruded cylindrical catalyst. This catalyst was named catalyst B.

The catalyst B was a product which does not contain at least one of an aluminum oxide and an aluminum hydroxide as the third component, and the amount of an alkali metal compound in terms of alkali metal was 0.014% by mass.

In the catalyst B, the Si/Al molar ratio of the first component was 450. Also, the content rate of the second component to the first component was 2.0% by mass in terms of alkaline earth metal, and the content rate of the methyl silicate to the first component was 20% by mass. As described above, the methyl silicate does not contain at least one of an aluminum oxide and an aluminum hydroxide.

### <Preparation Example 3>

An ammonium-type silicalite powder having an Si/Al molar ratio of 360 and having an MFI structure was obtained in the same manner as in Preparation Example 1, except that composition of the aqueous gel of Preparation Example 1 was changed. Its average particle diameter was 1 µm.

A proton-type silicalite powder was obtained by calcination of the thus obtained ammonium-type silicalite at 550°C for 6 hours under air flow. This catalyst was named catalyst C. In the catalyst C, the amount of the alkali metal compound in terms of alkali metal was 0.011% by mass.

In the catalyst C, the content rate of the second component to the first component was 0.0% by mass in terms of alkaline earth metal, and the content rate of the third component to the first component was 0% by mass.

### <Preparation Example 4>

An extruded cylindrical catalyst was obtained by the same method of Preparation Example 1, except that the ammonium-type silicalite powder obtained in Preparation Example 3 was used. This catalyst was named catalyst D. In the catalyst D, the amount of the alkali metal compound in terms of alkali metal was 0.026% by mass.

In the catalyst D, the Si/Al molar ratio of the first component was 360. Also, the content rate of the second component to the first component was 2.0% by mass in terms of alkaline earth metal, and the content rate of the third component to the first component was 25% by mass.

### <Preparation Example 5>

An extruded cylindrical catalyst was obtained by the same method of Preparation Example 4, except that calcium carbonate was not added. This catalyst was named catalyst E. In this catalyst E, the amount of the alkali metal compound in terms of alkali metal was 0.025% by mass.

In the catalyst E, the Si/Al molar ratio of the first component was 360. Also, the content rate of the second component to the first component was 0.0% by mass in terms of alkaline earth metal, and the content rate of the third component to the first component was 25% by mass.

### <Preparation Example 6>

An ammonium-type zeolite powder having an Si/Al molar ratio of 75 and having an MFI structure was obtained by the same method of Preparation Example 1, except that composition of the aqueous gel of Preparation Example 1 was changed. Its average particle diameter was 1 µm.

An extruded cylindrical catalyst was obtained by the same method of Preparation Example 1, except that this ammonium-type silicalite powder was used as the ammonium-type zeolite powder. This catalyst was named catalyst F. In this catalyst F, the amount of the alkali metal compound in terms of alkali metal was 0.028% by mass.

In the catalyst F, the mole ratio of Si/Al in the first component is 75, the content rate of the second component to the first component was 2.0% by mass in terms of alkaline earth metal, and the content rate of the third component to the first component was 25% by mass.

### <Preparation Example 7>

An extruded cylindrical catalyst was obtained by the same method of Preparation Example 4, except that the adding amount of the calcium carbonate was changed to 1.0g. This catalyst was named catalyst G. In the catalyst G, the content rate of the calcium carbonate as the second component to the first component was 0.4% by mass in terms of alkaline earth metal, the content rate of Al₂O₃ as the third component to the first component was 25% by mass, and the amount of the alkali metal compound in terms of alkali metal was 0.026% by mass.

### <Preparation Example 8>

An extruded cylindrical catalyst was obtained by the same method of Preparation Example 4, except that the adding amount of the calcium carbonate was changed to 10.0g. This catalyst was named catalyst H. In the catalyst H, the content rate of the calcium carbonate as the second component to the first component was 4.0% by mass in terms of alkaline earth metal, the content rate of Al₂O₃ as the third component to the first component was 25% by mass, and the amount of the alkali metal compound in terms of alkali metal was 0.026% by mass.

### <Preparation Example 9>

An extruded cylindrical catalyst was obtained by the same method of Preparation Example 4, except that the adding amount of the calcium carbonate was changed to 15.0g. This catalyst was named catalyst I. In the catalyst I, the content rate of the calcium carbonate as the second component to the first component was 6.0% by mass in terms of alkaline earth metal, the content rate of Al₂O₃ as the third component to the first component was 25% by mass, and the amount of the alkali metal compound in terms of alkali metal was 0.026% by mass.

### <Preparation Example 10>

An extruded cylindrical catalyst was obtained by the same method of Preparation Example 4, except that the calcium carbonate having an average particle diameter of 0.15 µm was used. This catalyst was named catalyst J. In the catalyst J, the content rate of the calcium carbonate as the second component to the first component was 2.0% by mass in terms of alkaline earth metal, the content rate of Al₂O₃ as the third component to the first component was 25% by mass, and the amount of the alkali metal compound in terms of alkali metal was 0.026% by mass.

### [Production of propylene]

The following shows examples and comparative examples of the production of propylene using the catalysts obtained in the above-mentioned preparation examples.

### <Comparative Example 1>

Steam treatment was carried out by exposing the catalyst F to an atmosphere of 530°C in temperature and of 0.08 MPa in steam partial pressure and 0.02 MPa in nitrogen partial pressure (each in absolute pressure) for 24 hours. Production of propylene was carried out using the steam-treated catalyst F.

A mixture of 125 mg of the aforementioned steam-treated catalyst F, which had been crushed into 0.5 to 1.0 mm, and 500 mg of quartz sand as a dilution agent was packed into a fixed-bed reactor of 6 mm in inner diameter, a reaction raw material which had been adjusted to a methanol concentration of 45.4% by mol, a 1-butene concentration of 27.3% by mol and an n-butane concentration of 27.3% by mol was passed through the reactor at an olefin-based WHSV of 2.15 Hr⁻¹ and the reaction was carried out at 550°C under normal pressure. A gas chromatography was used in the analysis of the reactor outlet gas.

Methanol was not observed in the reactor outlet gas at an early stage of the reaction, but methanol was observed after a lapse of a certain period of time. Thus, a lapse of time from the start of the reaction until methanol was observed in the reactor outlet gas and its conversion of methanol became less than 99.0% was defined as "catalyst life". The catalyst life of this Comparative Example 1 was regarded as 100.

### <Example 1>

Steam treatment was carried out by exposing the catalyst A to an atmosphere of 530°C in temperature and of 0.08 MPa in steam partial pressure and 0.02 MPa in nitrogen partial pressure (each in absolute pressure) for 24 hours.

Relative catalyst life was examined by carrying out production of propylene under the same conditions of Comparative Example 1, except that steam-treated catalyst A was used instead of steam-treated catalyst F. The results are shown in Table 1.

The relative catalyst life is a relative value of catalyst life in this example when the catalyst life in Comparative Example 1 was regarded as 100.

### <Example 2>

Steam treatment was carried out by exposing the catalyst D to an atmosphere of 530°C in temperature and of 0.08 MPa in steam partial pressure and 0.02 MPa in nitrogen partial pressure (each in absolute pressure) for 24 hours.

Relative catalyst life was examined by carrying out production of propylene under the same conditions of Comparative Example 1, except that steam-treated catalyst D was used instead of steam-treated catalyst F. The results are shown in Table 1.

### <Example 3>

Steam treatment was carried out by exposing the catalyst G to an atmosphere of 530°C in temperature and of 0.08 MPa in steam partial pressure and 0.02 MPa in nitrogen partial pressure (each in absolute pressure) for 24 hours.

Relative catalyst life was examined by carrying out production of propylene under the same conditions of Comparative Example 1, except that steam-treated catalyst G was used instead of steam-treated catalyst F. The results are shown in Table 1.

### <Example 4>

Steam treatment was carried out by exposing the catalyst H to an atmosphere of 530°C in temperature and of 0.08 MPa in steam partial pressure and 0.02 MPa in nitrogen partial pressure (each in absolute pressure) for 24 hours.

Relative catalyst life was examined by carrying out production of propylene under the same conditions of Comparative Example 1, except that steam-treated catalyst H was used instead of steam-treated catalyst F. The results are shown in Table 1.

### <Example 5>

Steam treatment was carried out by exposing the catalyst I to an atmosphere of 530°C in temperature and of 0.08 MPa in steam partial pressure and 0.02 MPa in nitrogen partial pressure (each in absolute pressure) for 24 hours.

Relative catalyst life was examined by carrying out production of propylene under the same conditions of Comparative Example 1, except that steam-treated catalyst I was used instead of steam-treated catalyst F. The results are shown in Table 1.

### <Example 6>

Steam treatment was carried out by exposing the catalyst J to an atmosphere of 530°C in temperature and of 0.08 MPa in steam partial pressure and 0.02 MPa in nitrogen partial pressure (each in absolute pressure) for 24 hours.

Relative catalyst life was examined by carrying out production of propylene under the same conditions of Comparative Example 1, except that steam-treated catalyst J was used instead of steam-treated catalyst F. The results are shown in Table 1.

### <Comparative Example 2>

Steam treatment was carried out by exposing the catalyst E to an atmosphere of 530°C in temperature and of 0.08 MPa in steam partial pressure and 0.02 MPa in nitrogen partial pressure (each in absolute pressure) for 24 hours.

Relative catalyst life was examined by carrying out production of propylene under the same conditions of Comparative Example 1, except that steam-treated catalyst E was used instead of catalyst F. The results are shown in Table 1.

### <Comparative Example 3>

Relative catalyst life was examined by carrying out production of propylene under the same conditions of Comparative Example 1, except that the catalyst C was used instead of catalyst F. The results are shown in Table 1.

### [Table 1]

**Table 1**

| | Catalyst | First component Si/Al molar ratio | Second component content (% by mass) | Third component content (% by mass) | Relative life |
|---|---|---|---|---|---|
| Comparative Example 1 | F (Preparation Example 6) | 75 | 2.0 | 25 | 100 |
| Example 1 | A (Preparation Example 1) | 450 | 2.0 | 25 | 144 |
| Example 2 | D (Preparation Example 4) | 360 | 2.0 | 25 | 125 |
| Example 3 | G (Preparation Example 7) | 360 | 0.4 | 25 | 118 |
| Example 4 | H (Preparation Example 8) | 360 | 4.0 | 25 | 133 |
| Example 5 | I (Preparation Example 9) | 360 | 6.0 | 25 | 141 |
| Example 6 | J (Preparation Example 10) | 360 | 2.0 | 25 | 147 |
| Comparative Example 2 | E (Preparation Example 5) | 360 | 0.0 | 25 | 70 |
| Comparative Example 3 | C (Preparation Example 3) | 360 | 0.0 | 0 | 77 |

As shown in Table 1, it was found that the catalyst life is improved by the use of the catalyst A, catalyst D, catalyst G, catalyst H, catalyst I and catalyst J in which the Si/Al molar ratio of the first component is more than 300.

In addition, it was found that the catalyst life is improved by the use of the catalyst D which contains all of the first component, second component and third component, in comparison with the catalyst E from which the second component was excluded and the catalyst C consisting of only the first component.

### <Example 7>

Production of propylene was carried out using the catalyst A. A mixture of 3.5 g of the catalyst A, which had been crushed into 0.5 to 0.6 mm, and 3.5 g of quartz sand as a dilution agent was packed into a fixed-bed reactor of 12 mm in inner diameter, a reaction raw material which had been adjusted to a methanol concentration of 11.1% by mol, a dimethyl ether concentration of 22.2% by mol, an isobutene concentration of 22.9% by mol, a steam concentration of 22.2% by mol and a nitrogen concentration of 21.6% by mol was passed through the reactor at an olefin-based WHSV of 6.0 Hr⁻¹ and the reaction was carried out at 540°C under 0.23 MPa (absolute pressure). A gas chromatography was used in the analysis of the reactor outlet gas.

Methanol and dimethyl ether were not observed in the reactor outlet gas at an early stage of the reaction, but these oxygen-containing compounds were observed after a lapse of a certain period of time. Thus, a lapse of time from the start of the reaction until these oxygen-containing compounds were observed in the reactor outlet gas and conversion of the oxygen-containing compounds became less than 99.0% was defined as "catalyst life".

In this connection, the conversion of oxygen-containing compound is a value calculated by ((mol flow rate of methanol at the reactor inlet) + (mol flow rate of dimethyl ether at the reactor inlet) × 2 - (mol flow rate of methanol at the reactor outlet) - (mol flow rate of dimethyl ether at the reactor outlet) × 2))/((mol flow rate of methanol at the reactor inlet) + (mol flow rate of dimethyl ether at the reactor inlet) × 2) × 100.

When the conversion of the oxygen-containing compound became less than 99.0% in the first catalyst life evaluation (Run 1), supply of the material was stopped and regeneration of the catalyst was carried out at 550°C for 24 hours under atomospheric pressure by passing an oxygen-containing regeneration gas which had been adjusted to 1% by mol of oxygen and 99% by mol of nitrogen, through the catalyst bed in the reactor at a rate of 5 Nm³/hr·kg-catalyst.

By supplying the material to the catalyst after regeneration in the same manner as in the above, the second catalyst life evaluation (Run 2) was carried out under the same conditions of the reaction conditions of the first catalyst life evaluation (Run 1). After the Run 2, the regeneration of catalyst was carried out until Run 5 by repeating it in the same manner as in Run 1.

In this connection, in and after the Run 3, steam treatment was carried out before supplying the reaction raw material, by exposing the catalyst after regeneration to an atmosphere of 530°C in temperature and of 0.08 MPa in steam partial pressure and 0.02 MPa in nitrogen partial pressure (each in absolute pressure) for 24 hours.

Relative catalyst lives of Runs 1 to 5 are shown in Table 2. The term "relative catalyst life" as used herein means a relative value when the catalyst life of Run 1 of this Example 7 is regarded as 100.

### <Comparative Example 4>

Production of propylene was carried out under the same conditions of Example 7, except that the catalyst B was used instead of the catalyst A. Relative catalyst lives of Runs 1 to 5 are shown in Table 2.

### [Table 2]

**Table 2**

| | Catalyst | Si/Al molar ratio | Relative life | | | | | |
|---|---|---|---|---|---|---|---|---|
| | | | Run 1 | Run 2 | Run 3 | Run 4 | Run 5 | Total |
| Example 7 | A (Preparation Example 1) | 450 | 100 | 64 | 103 | 123 | 109 | 499 |
| Comparative Example 4 | B (Preparation Example 2) | 450 | 99 | 98 | 90 | 76 | 68 | 431 |

As shown in Table 2, the life of the catalyst B which does not contain aluminum (hydro)oxide as the third component was shortened as the life evaluation was repeated. Contrary to this, it was found that the catalyst A which contains aluminum (hydro)oxide can be used stably for a prolonged period of time because its life was not shortened when the life evaluation was repeated.

In addition, since the lives of in and after Run 3 of Example 7 are longer than the lives of Run 1 and Run 2, it was found that the catalyst life is improved by the steam treatment before the reaction.

### [Catalyst activity]

The following shows activities of the catalysts obtained by the aforementioned preparation examples as reference examples.

### <Reference Example 1>

Steam treatment was carried out by exposing the catalyst A to an atmosphere of 530°C in temperature and of 0.08 MPa in steam partial pressure and 0.02 MPa in nitrogen partial pressure (each in absolute pressure) for 24 hours.

The steam-treated catalyst A was packed into a fixed-bed reactor of 28 mm in inner diameter, a material which had been adjusted to a dimethyl ether concentration of 29.4% by mol, a 1-butene concentration of 20.6% by mol and a nitrogen concentration of 50.0% by mol was passed through the reactor at an olefin-based WHSV of 6.0 Hr⁻¹ and the reaction was carried out at 550°C under 0.30 MPa (absolute pressure). A gas chromatography was used in the analysis of the reactor outlet gas.

The conversion of dimethyl ether and conversion of 1-butene are shown in Table 3.

In this connection, the conversion of dimethyl ether is a value calculated by ((mol flow rate of dimethyl ether at the reactor inlet) × 2 - (mol flow rate of methanol at the reactor outlet) - (mol flow rate of dimethyl ether at the reactor outlet) × 2))/((mol flow rate of dimethyl ether at the reactor inlet) × 2) × 100, and the conversion of 1-butene is a value calculated by ((mol flow rate of 1-butene at the reactor inlet) - (total mol flow rate of butene isomers at the reactor outlet))/(mol flow rate of 1-butene at the reactor inlet) × 100.

### <Reference Examples 2 to 4>

Respective reactions were carried out under the same conditions of Reference Example 1, except that the catalysts B to D were used instead of the catalyst A. The conversion of dimethyl ether and conversion of 1-butene are shown in Table 3.

**Table 3**

| | Catalyst | Si/Al molar ratio | Conversion | |
|---|---|---|---|---|
| | | | Dimethyl ether | 1-Butene |
| Reference Example 1 | A (Preparation Example 1) | 450 | 100 | 57.1 |
| Reference Example 2 | B (Preparation Example 2) | 450 | 100 | 57.4 |
| Reference Example 3 | D (Preparation Example 4) | 360 | 100 | 56.7 |
| Reference Example 4 | F (Preparation Example 6) | 75 | 100 | 59.2 |

As shown in Table 3, it was found that, under a condition of coexisting an olefin having 4 or more carbon atoms, the conversion of the reaction was not greatly different from the conversion of the catalyst D having an Si/Al molar ratio of 75, even when the catalysts A to C having an Si/Al molar ratio of more than 300 were used.

<Reference Example 5>

Production of propylene was carried out using the catalyst A and using methanol as the material. A mixture of 5 mg of the catalyst A, which had been crushed into 0.5 to 0.6 mm, and 500 mg of quartz sand as a dilution agent was packed into a fixed-bed reactor of 6 mm in inner diameter, a reaction raw material which had been adjusted to a methanol concentration of 6.5% by mol, a steam concentration of 26.0% by mol and a nitrogen concentration of 67.5% by mol was passed through the reactor at a methanol WHSV of 10.0 Hr⁻¹ and the reaction was carried out at 550°C under 0.10 MPa (absolute pressure). A gas chromatography was used in the analysis of the reactor outlet gas. As a result, the methanol conversion was 1.9%.

In this connection, the conversion of methanol is a value calculated by ((mol flow rate of methanol at the reactor inlet) - (mol flow rate of methanol at the reactor outlet) - (mol flow rate of dimethyl ether at the reactor outlet) × 2))/(mol flow rate of methanol at the reactor inlet) × 100.

### <Reference Example 6>

Production of propylene from methanol and butene as the materials was carried out using the catalyst A. This was carried out under the same conditions of Reference Example 5, except that the nitrogen concentration was lowered instead of the introduction of butene in Reference Example 5. That is, a reaction raw material which had been adjusted to a methanol concentration of 6.5% by mol, a trans-2-butene concentration of 6.5% by mol, a steam concentration of 26.0% by mol and a nitrogen concentration of 61.0% by mol was passed through the reactor at a methanol WHSV of 10.0 Hr⁻¹ and the reaction was carried out at 550°C under 0.10 MPa (absolute pressure). A gas chromatography was used in the analysis of the reactor outlet gas. As a result, the conversion of methanol was 95.5%.

From the comparison of Reference Examples 5 and 6, it was found that by the presence of an olefin having 4 or more carbon atoms, the reaction rate becomes high and the catalyst A having extremely small amount of Al shows sufficient catalyst activity.

While the invention has been described in detail and with reference to specific embodiments thereof, it will be apparent to one skilled in the art that various changes and modifications can be made therein without departing from the scope of the invention as defined in the appended claims.

## Claims

1. A method for manufacturing propylene, which comprises, as a reaction step, bringing an oxygen-containing compound into contact with a catalyst in the presence of an olefin having 4 or more carbon atoms to manufacture propylene,
wherein the catalyst is a composite comprising:
a proton-type silicalite as a first component;
an alkaline earth metal compound, as a second component; and
a non-zeolite compound comprising an aluminum oxide, as a third component, wherein
a molar ratio of Si/Al in the first component is more than 300,
a content rate of the second component to the first component is 0.1% by mass or more in terms of alkaline earth metal, and
a content rate of the third component in terms of Al2O3 to the first component is 5% to 50% by mass.

2. The method for manufacturing propylene according to claim 1, wherein the first component is a silicalite comprising an MFI structure.

3. The method for manufacturing propylene according to claim 1 or 2, wherein the second component is a calcium compound.

4. The method for manufacturing propylene according to any one of claims 1 to 3, wherein the oxygen-containing compound is at least one of methanol and dimethyl ether.

5. The method for manufacturing propylene according to any one of claims 1 to 4, which comprises bringing the catalyst into contact with a steam atmosphere before the reaction step.

6. The method for manufacturing propylene according to any one of claims 1 to 5, which comprises bringing the catalyst, to which a coke used in the reaction step is adhered, into contact with an oxygen-containing gas to remove the coke to regenerate the catalyst, wherein the regenerated catalyst is supplied to the reaction step.

7. The method for manufacturing propylene according to claim 6, which comprises bringing the regenerated catalyst into contact with a steam atmosphere before the regenerated catalyst is supplied to the reaction step.

8. A catalyst for manufacturing propylene by bringing into contact with an oxygen-containing compound in the presence of an olefin having 4 or more carbon atoms, wherein the catalyst is a composite comprising:
a proton-type silicalite as a first component;
an alkaline earth metal compound, as a second component; and
a non-zeolite compound comprising an aluminum oxide, as a third component, wherein
a molar ratio of Si/Al in the first component is more than 300,
a content rate of the second component to the first component is 0.1% by mass or more in terms of alkaline earth metal, and
a content rate of the third component in terms of Al2O3 to the first component is 5% to 50% by mass.

9. The catalyst for manufacturing propylene according to claim 8, wherein the first component is a silicalite comprising an MFI structure.

10. The catalyst for manufacturing propylene according to claim 8 or 9, wherein the second component is a calcium compound.

11. The method or the catalyst for manufacturing propylene according to any one of the preceding claims, wherein a content rate of the second component to the first component is 0.1% to 20% by mass in terms of alkaline earth metal.

12. The method or the catalyst for manufacturing propylene according to any one of the preceding claims, wherein a molar ratio of Si/Al in the first component is more than 300 and 1000 or less.

## Patentansprüche

1. Ein Verfahren zur Herstellung von Propylen, umfassend als einen Reaktionsschritt das in Kontakt bringen einer sauerstoffhaltigen Verbindung mit einem Katalysator in Gegenwart eines Olefins mit 4 oder mehr Kohlenstoffatomen, um Propylen herzustellen,
wobei der Katalysator ein Verbundstoff ist, umfassend:
ein Silicalit vom Protonentyp als eine erste Komponente;
eine Erdalkalimetallverbindung als eine zweite Komponente; und
eine Nicht-Zeolith-Verbindung, umfassend ein Aluminiumoxid, als eine dritte Komponente,
wobei
ein Molverhältnis von Si/Al in der ersten Komponente mehr als 300 beträgt,
ein Gehaltsverhältnis der zweiten Komponente zur ersten Komponente 0,1 Massen-% oder mehr, bezogen auf das Erdalkalimetall, beträgt und
ein Gehaltsverhältnis der dritten Komponente, bezogen auf Al₂O₃, zur ersten Komponente 5 bis 50 Massen-% beträgt.

2. Das Verfahren zur Herstellung von Propylen gemäß Anspruch 1, wobei die erste Komponente ein Silicalit, umfassend eine MFI Struktur, ist.

3. Das Verfahren zur Herstellung von Propylen gemäß Anspruch 1 oder 2, wobei die zweite Komponente eine Calciumverbindung ist.

4. Das Verfahren zur Herstellung von Propylen gemäß einem der Ansprüche 1 bis 3, wobei die sauerstoffhaltige Verbindung mindestens eine aus Methanol und Dimethylether ist.

5. Das Verfahren zur Herstellung von Propylen gemäß einem der Ansprüche 1 bis 4, welches das in Kontakt bringen des Katalysators mit einer Dampfatmosphäre vor dem Reaktionsschritt umfasst.

6. Das Verfahren zur Herstellung von Propylen gemäß einem der Ansprüche 1 bis 5, welches das in Kontakt bringen des Katalysators, an dem ein Koks haftet, der im Reaktionsschritt verwendet wird, mit einem sauerstoffhaltigen Gas umfasst, um den Koks zu entfernen, um den Katalysator zu regenerieren, wobei der regenerierte Katalysator dem Reaktionsschritt zugeführt wird.

7. Das Verfahren zur Herstellung von Propylen gemäß Anspruch 6, welches das in Kontakt bringen des regenerierten Katalysators mit einer Dampfatmosphäre umfasst, bevor der regenerierte Katalysator dem Reaktionsschritt zugeführt wird.

8. Ein Katalysator zur Herstellung von Propylen durch in Kontakt bringen mit einer sauerstoffhaltigen Verbindung in Gegenwart eines Olefins mit 4 oder mehr Kohlenstoffatomen, wobei der Katalysator ein Verbundstoff ist, umfassend:
ein Silicalit vom Protonentyp als eine erste Komponente;
eine Erdalkalimetallverbindung als eine zweite Komponente; und
eine Nicht-Zeolith-Verbindung, umfassend ein Aluminiumoxid, als eine dritte Komponente,
wobei
ein Molverhältnis von Si/Al in der ersten Komponente mehr als 300 beträgt,
ein Gehaltsverhältnis der zweiten Komponente zur ersten Komponente 0,1 Massen-% oder mehr, bezogen auf das Erdalkalimetall, beträgt und
ein Gehaltsverhältnis der dritten Komponente, bezogen auf Al₂O₃, zur ersten Komponente 5 bis 50 Massen-% beträgt.

9. Der Katalysator zur Herstellung von Propylen gemäß Anspruch 8, wobei die erste Komponente ein Silicalit, umfassend eine MFI Struktur, ist.

10. Der Katalysator zur Herstellung von Propylen gemäß Anspruch 8 oder 9, wobei die zweite Komponente eine Calciumverbindung ist.

11. Das Verfahren oder der Katalysator zur Herstellung von Propylen gemäß einem der vorhergehenden Ansprüche, wobei ein Gehaltsverhältnis der zweiten Komponente zur ersten Komponente 0,1 bis 20 Massen-%, bezogen auf das Erdalkalimetall, beträgt.

12. Das Verfahren oder der Katalysator zur Herstellung von Propylen gemäß einem der vorhergehenden Ansprüche, wobei ein Molverhältnis von Si/Al in der ersten Komponente mehr als 300 und 1000 oder weniger beträgt.

## Revendications

1. Méthode de fabrication de propylène qui comprend, en tant qu'étape réactionnelle, la mise en contact d'un composé contenant de l'oxygène avec un catalyseur en présence d'une oléfine ayant 4 atomes de carbone ou plus pour fabriquer du propylène,
dans laquelle le catalyseur est un composite comprenant :
un silicalite de type proton en tant que premier composant ;
un composé de métal alcalino-terreux en tant que deuxième composant ; et
un composé non-zéolite comprenant un oxyde d'aluminium en tant que troisième composant,
dans laquelle
le rapport molaire Si/Al dans le premier composant est supérieur à 300,
le taux en contenu du deuxième composant par rapport au premier composant est de 0,1 % en masse ou plus en termes de métal alcalino-terreux, et
le taux en contenu du troisième composant en termes d'Al₂O₃ par rapport au premier composant est de 5 % à 50 % en masse.

2. Méthode de fabrication de propylène selon la revendication 1, dans laquelle le premier composant est un silicalite comprenant une structure MFI.

3. Méthode de fabrication de propylène selon la revendication 1 ou 2, dans laquelle le deuxième composant est un composé calcium.

4. Méthode de fabrication de propylène selon l'une quelconque des revendications 1 à 3, dans laquelle le composé contenant de l'oxygène est au moins l'un parmi le méthanol et le diméthyléther.

5. Méthode de fabrication de propylène selon l'une quelconque des revendications 1 à 4, qui comprend la mise en contact du catalyseur avec une atmosphère de vapeur avant l'étape réactionnelle.

6. Méthode de fabrication de propylène selon l'une quelconque des revendications 1 à 5, qui comprend la mise en contact du catalyseur, auquel adhère un coke utilisé dans l'étape réactionnelle, avec un gaz contenant de l'oxygène pour éliminer le coke pour régénérer le catalyseur, dans laquelle le catalyseur régénéré est fourni à l'étape réactionnelle.

7. Méthode de fabrication de propylène selon la revendication 6, qui comprend la mise en contact du catalyseur régénéré avec une atmosphère de vapeur avant que le catalyseur régénéré soit fourni à l'étape réactionnelle.

8. Catalyseur pour fabriquer du propylène par mise en contact avec un composé contenant de l'oxygène en présence d'une oléfine ayant 4 atomes de carbone ou plus, lequel catalyseur est un composite comprenant :
un silicalite de type proton en tant que premier composant ;
un composé de métal alcalino-terreux en tant que deuxième composant ; et
un composé non-zéolite comprenant un oxyde d'aluminium en tant que troisième composant,
dans lequel
le rapport molaire Si/Al dans le premier composant est supérieur à 300,
le taux en contenu du deuxième composant par rapport au premier composant est de 0,1 % en masse ou plus en termes de métal alcalino-terreux, et
le taux en contenu du troisième composant en termes d'Al₂O₃ par rapport au premier composant est de 5 % à 50 % en masse.

9. Catalyseur pour fabriquer du propylène selon la revendication 8, dans lequel le premier composant est un silicalite comprenant une structure MFI.

10. Catalyseur pour fabriquer du propylène selon la revendication 8 ou 9, dans lequel le deuxième composant est un composé calcium.

11. Méthode ou catalyseur pour fabriquer du propylène selon l'une quelconque des revendications précédentes, dans lequel le taux en contenu du deuxième composant par rapport au premier composant est de 0,1 % à 20 % en masse en termes de métal alcalino-terreux.

12. Méthode ou catalyseur pour fabriquer du propylène selon l'une quelconque des revendications précédentes, dans lequel le rapport molaire Si/Al dans le premier composant est supérieur à 300 et de 1000 ou moins.
